# EUROPEAN PATENT SPECIFICATION

(11) **EP 3 765 437 B1**
(45) Date of publication and mention of the grant of the patent: **20.12.2023**
(21) Application number: 19709949.2
(22) Date of filing: 12.03.2019
(51) Int. Cl.: C07C 227/38, C07C 227/40, C07C 227/44, A23K 50/75, A23K 50/30, A23K 20/142, B01D 61/44, B01D 61/42

(54) **METHODS OF STABILIZING AND PURIFYING AQUEOUS COMPOSITIONS OF ALKALI METAL SALTS OF AMINO ACIDS COMPRISING ALKALI METAL HYDROXIDE**
VERFAHREN ZUR STABILISIERUNG UND REINIGUNG VON WÄSSRIGEN ZUSAMMENSETZUNGEN VON ALKALIMETALLSALZEN VON AMINOSÄUREN MIT ALKALIMETALLHYDROXID
PROCÉDÉS DE STABILISATION ET DE PURIFICATION DE COMPOSITIONS AQUEUSES DE SELS DE MÉTAUX ALCALINS D'ACIDES AMINÉS COMPRENANT UN HYDROXYDE DE MÉTAL ALCALIN

(30) Priority: 13.03.2018 EP 18161605
(43) Date of publication of application: 20.01.2021
(73) Proprietor: Taminco BV, 9000 Gent (BE)
(72) Inventor: MOONEN, Kristof, 9220 Hamme (BE); DUMOLEIJN, Kim, 4529 GW Eede (NL); SCHELDEMAN, Daan, 8790 Waregem (BE); ULRICHTS, Dieter Koenraad Roel, 8200 Sint-Adries (BE); PINOY, Luc Joseph Jan, 9040 Sint-Amandsberg (BE); LAUWAERTS, Angelo Hermes Johannes, 9000 Gent (BE)
(74) Representative: Ricker, Mathias
(86) International application number: PCT/EP2019/056165
(87) International publication number: WO 2019/175175

(56) References cited:
- WO-A1-2007/107184
- WO-A1-2009/016025
- WO-A1-2013/174764
- DE-A1-102006 016 227
- US-A- 4 258 210
- Marcello Fidaleo ET AL: "Electrodialysis Applications in The Food Industry" In: "Advances in Food and Nutrition Research Volume 67", 1 January 2006 (2006-01-01), Elsevier, XP055193295, ISSN: 1043-4526 ISBN: 978-0-12-394598-3 vol. 51, pages 265-360, DOI: 10.1016/S1043-4526(06)51005-8,

## Description

### FIELD OF THE INVENTION

The invention relates to a method of stabilizing and/or purifying a composition comprising an alkali metal salt of a compound of the general formula R¹R²N-CHR³-COOH or R¹R²N-CHR³CHR³-COOH, in particular the sodium salt of N,N-dimethylglycine or the sodium salt of β-alanine, to the purified composition as such, and to the use thereof.

### BACKGROUND OF THE INVENTION

It is known to use specific amino acids or salts thereof as a feed additive.

WO 2007107184 A1 discloses an alkali metal salt of a compound of the general formula R¹R²N-CH₂-COOH, wherein R¹ and R² are independently an alkyl, an alkenyl, or a hydroxyalkyl radical containing from 1 to 18 carbon atoms, or from 1 to 6 carbon atoms, or wherein R¹ and R² form jointly together with the N atom a heterocyclic 5- or 6-membered ring, such as the sodium salt of amino acid N,N-dimethylglycine (Na-DMG). The salt may be used as a feed additive for poultry.

WO 2013174764 A1 discloses a compound of the general formula R¹R²N-CH₂-CH₂-COOH, wherein R¹ and R² are independently hydrogen, acetyl or a straight or branched alkyl radical containing from 1 to 4 carbon atoms or salts thereof, such as β-alanine. This compound may be used as a feed additive for poultry, pigs or fish. WO 2009/016025 addresses the presence of impurities contaminating Na-β-alaninate from β-amino-propionitrile. US 4 258 210 discloses a similar process like WO 2009/016025. DE 10 2006 016227 and Marcello Fidaleo et al. "Electrodialysis Applications in the food industry", Advances in Food an Nutrition Research Vol. 67, January 1, 2006, Vol. 51, pages 265-360) both relate to compositions of Na-DMG, their use and their manufacturing process.

As is known in the art, N,N-dimethyl glycine (DMG) can be manufactured via at least three routes, each requiring the use of a strong base, e.g. sodium hydroxide, and hence resulting in the corresponding glycinate salt.

According to a first route, formaldehyde, dimethylamine (DMA) and HCN or a salt thereof are reacted in a Strecker reaction upon formation of dimethyl amino acetonitrile (DMAAN). The intermediate DMAAN is then hydrolyzed in hot aqueous NaOH solution or another suitable strong base to obtain Na-DMG and ammonia as co-product. Relevant side reactions are formaldehyde forming reactions, i.e. essentially the backward reaction of the Strecker reaction, hexamethylenetetramine (HMTA) formation reaction from formaldehyde and NH₃, and cyanide forming reactions.

Similarly, β-alanine salts can be formed by hydrolysis of 3-amino propionitrile in a hot aqueous NaOH solution or another suitable base. 3-amino propionitrile can be produced by reaction of acrylonitrile and ammonia.

According to a second route, dimethylamine (DMA) is alkylated with monochloro acetic acid (MCA) yielding DMG. MCA, however, is an acid and hence will also consume one equivalent of DMA as a base to neutralize. Hence, the obtained product is dimethylammonium N,N-dimethylglycinate instead of the sodium salt. Strong base such as NaOH is used to liberate valuable dimethylamine as a vapor from the salt and replace it with the counterion of the strong base such as Na⁺.
According to a third route, DMG can be obtained from N,N-dimethyl amino ethanol (DMAE or alkyl alkanolamine AAA) by converting the alcohol moiety in DMAE into a carboxylic acid moiety. This reaction can be done by either an oxidation reaction by means of the addition of an oxidant, e.g. O₂, or by a dehydrogenation reaction by means of abstraction of hydrogen. Both processes can only occur under the action of a catalyst, usually a metal catalyst. Both catalytic routes require neutralization of the obtained carboxylic acid in order to keep the pH high in the range where the catalyst is active. Therefore, in practice, excess NaOH or any other strong base needs to be added to the liquid reaction medium of such a process. The reaction product commonly contains non-reacted DMAE (AAA).

Generally, excess of the strong base, e.g. NaOH, needs to be used in the above synthesis protocols in order to obtain high conversion of starting materials or to avoid the formation of side products. After full conversions have been reached, the excess of unconverted strong base resides in the amino acid salt solution.

If aqueous solutions of sodium N,N-dimethylglycinate prepared according to the methods known from the prior art are concentrated at higher temperature or the mixtures are dried in order to obtain Na-DMG e.g. in powder or granular form, sticking of the metal salt of N,N-dimethylglycine may be seen during concentration or drying, and/or degradation of the salt.

The term "stickiness" as used herein means that particles of the powder or granulate adhere together, thus forming agglomerates. Stickiness may also be accompanied by gradually softening of the powder or granulate such that the flowability is deteriorated.

Furthermore, such concentrated and/or dried amino acid salts may be corrosive towards for instance eye or skin.

Also, the stability of the acid salts may be worsened. The term "stability of the salt is worsened" means that the amino acid salt may lose chemical and/or physical properties, e.g. during storage.

Stability may be e.g. negatively affected by the influence of increasing temperature. This means that under the influence of increasing temperature physical and/or chemical properties of the amino acid salt may change.

Increasing temperature may e.g. result in degradation, i.e. the amino acid salt changes its chemical structure.

Such negatively affected products are not usable as a feed additive.

Alkali metal salts of β-alanine (AL) may also be used as feed additive. Such metal salts may be produced by basic hydrolysis of 3-aminopropionitrile or oxidation of 3-aminopropanol. Similar problems as with alkali metal salts of DMG may arise here, too.

It would be desirable to provide a product not having one or more of the addressed drawbacks, i.e. to provide an improved product.

### SUMMARY OF THE INVENTION

The inventors discovered that the stickiness, corrosivity and/or degradation upon drying may be attributed to the presence of alkali metal hydroxide in the final product, e.g. sodium hydroxide in Na-DMG. Accordingly, improved products in terms of stabilization and/or purification may be obtained, when the quantity of alkali metal hydroxide in the product composition, e.g. in Na-DMG, is kept as low as possible. The inventors discovered that one or more of the addressed drawbacks can be reduced or even avoided when the quantity of alkali metal hydroxide in the final product composition is desirably below 0.5 % by weight, or below 0.1 % by weight, or below 0.05 % by weight, based on the weight of the alkali metal salt of the amino acid, e.g. Na-DMG, and the alkali metal hydroxide.

The inventors developed two different techniques (A) and (B) according to claims 1 and 2 which can be applied in order to reduce the metal hydroxide content in a mixture comprising alkali metal hydroxide, water and an alkali metal salt of an amino acid of the general formula (I) or formula (II)

R¹R²N-CHR³-COOH (I)

or

R¹R²N-CHR³CHR³-COOH (II).

Any one or a combination of the methods according to the invention result in a depletion of the quantity of alkali metal hydroxide in a product composition comprising the salt and the alkali metal hydroxide, if any remains, relative to the quantity of alkali metal hydroxide present in the feed mixture, and an enrichment in the concentration of the respective alkali metal salt in the product composition relative to the concentration of the respective alkali metal salt in the feed mixture composition.

Also claimed is a composition consisting of an alkali metal salt of a compound of the general formula (I) or formula (II) as defined in claims 9, 11 and the use of such a composition as feed additive as defined in claim 12.

### BRIEF DISCUSSION OF THE FIGURES

In the figures shows:

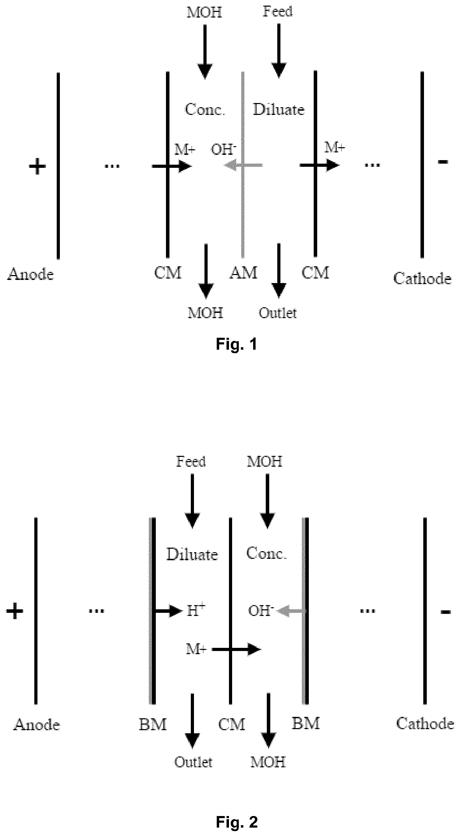

**Fig. 1****:** a schematic view of an electrodialysis cell comprising two compartments comprising one anion-selective membrane AM and one cationic membrane CM as a repeating unit (and forming a CM/AM/CM electrodialysis cell) suitable for removal of alkali metal hydroxide from a mixture of an alkali metal salt of a compound of the general formula (I) or formula (II), wherein said alkali metal salt is comprised in a mixture at least comprising said salt, an alkali metal hydroxide and water, according to a first embodiment (A1) of technique (A);
**Fig. 2****:** a schematic view of an electrodialysis cell comprising two compartments comprising one bipolar membrane BM and one cationic membrane CM as a repeating unit (and forming a BM/CM/BM electrodialysis cell) suitable for removal of alkali metal hydroxide from a mixture of an alkali metal salt of a compound of the general formula (I) or formula (II), wherein said alkali metal salt is comprised in a mixture at least comprising said salt, an alkali metal hydroxide and water, according to a second embodiment (A2) of technique (A);

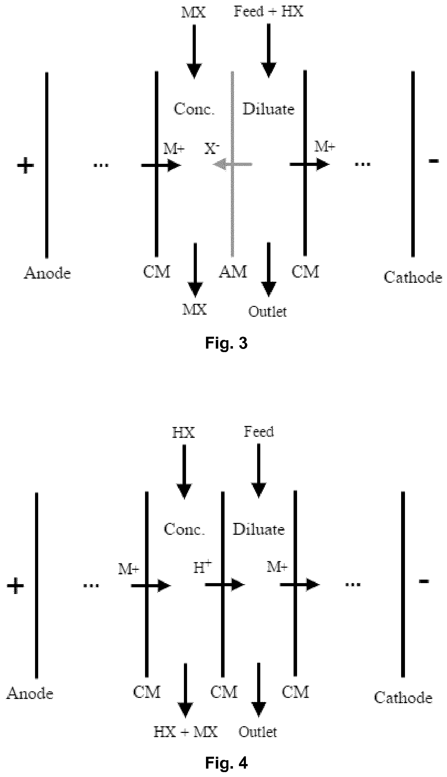

**Fig. 3****:** a schematic view of an electrodialysis cell comprising two compartments comprising one anionic membrane AM and one cationic membrane CM as a repeating unit (and forming a CM/AM/CM dialysis cell) suitable for desalination of a mixture of an alkali metal salt of a compound of the general formula (I) or formula (II), wherein said alkali metal salt is comprised in a mixture at least comprising said salt, an alkali metal hydroxide and water, and wherein said mixture has been treated with an acid prior to electrodialysis, according to a third embodiment (A3) of technique (A);
**Fig. 4****:** a schematic view of an electrodialysis cell comprising two compartments comprising two cationic membranes CM as a repeating unit (and forming a CM/CM/CM electrodialysis cell) for removal of alkali metal hydroxide from a compound of the general formula (I) or formula (II), wherein said alkali metal salt is comprised in a mixture at least comprising said salt, an alkali metal hydroxide and water, and wherein said mixture is treated with an acid simultaneously to electrodialysis, according to a fourth embodiment (A4) of technique (A);

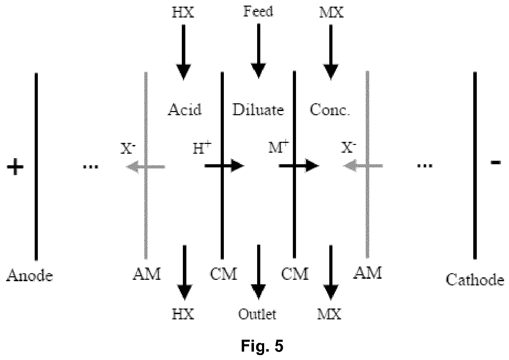

**Fig. 5****:** a schematic view of an electrodialysis cell comprising three compartments comprising one anionic membrane AM and two cationic membranes CM as a repeating unit (and forming a AM/CM/CM/AM electrodialyis cell) for removal of alkali metal hydroxide from a compound of the general formula (I) or formula (II), wherein said alkali metal salt is comprised in a mixture at least comprising said salt, an alkali metal hydroxide and water, and wherein said mixture is treated with an acid simultaneously to electrodialysis, according to a fifth embodiment (A5) of technique (A);

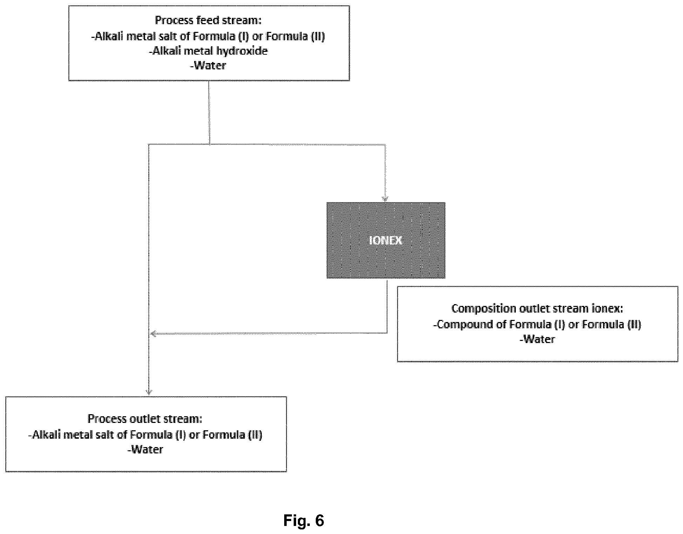

**Fig. 6****:** a schematic flow scheme in an arrangement for neutralizing alkali metal hydroxide in said mixture at least comprising said salt, alkali metal hydroxide and water, purifying a part of said mixture by subjecting it to an ion exchanger (lonex), and adding R¹R²N-CHR³-COOH or R¹R²N-CH₂CH₂-COOH obtained by said subjection to the residual stream of the mixture having not been subjected to ion exchange according to technique (B).

### DETAILED DESCRIPTION OF THE INVENTION

In a first aspect, the invention relates to a method of stabilizing an alkali metal salt of a compound of the general formula (I) or formula (II) as claimed in the claims

R¹R²N-CHR³-COOH (I)

or

R¹R²N-CHR³CHR³-COOH (II),

wherein R¹, R² and R³ are independently hydrogen, an alkyl, an alkenyl, or a hydroxyalkyl radical containing from 1 to 18 carbon atoms, or from 1 to 6 carbon atoms; or wherein R¹ and R² form jointly together with the N atom a heterocyclic 5- or 6-membered ring.

The invention further relates to a method of purifying an alkali metal salt of a compound of the general formula (I) or formula (II) as claimed in the claims

R¹R²N-CHR³-COOH (I)

or

R¹R²N-CHR³CHR³-COOH (II),

wherein R^{1,} R² and R³ are independently H, an alkyl, an alkenyl, or a hydroxyalkyl radical containing from 1 to 18 carbon atoms, or from 1 to 6 carbon atoms; or wherein R¹ and R² form jointly together with the N atom a heterocyclic 5- or 6-membered ring.

The term "method of stabilizing an alkali metal salt of a compound of the general formula (I) or formula (II)" may be synonymously used with terms such as "method of making an alkali metal salt of a compound of the general formula (I) or formula (II)" or "method of increasing the thermal stability of an alkali metal salt of a compound of the general formula (I) or formula (II)" or "method of reducing thermal degradation of an alkali metal salt of a compound of the general formula (I) or formula (II)" or "method of reducing stickiness of an alkali metal salt of a compound of the general formula (I) or formula (II)." This is since a method of stabilizing an alkali metal salt in the meaning of the invention at least encompasses a method of increasing the thermal stability of the alkali metal salt or a method of reducing the thermal degradation of said alkali metal salt or reducing the stickiness of said alkali metal salt, i.e. the invention aims at maintaining the chemical and physical properties of the amino acid salt such that it may be used as feed additive. The meaning of terms such as "stickiness" or "stability" is defined in the Background section above.

As disclosed in the Background section, said alkali metal salt of the compound of formula (I) or formula (II) can be prepared in a process for making a compound of formula (I) or formula (II) in the presence of an alkali metal hydroxide.

The alkali metal salt of the compound of formula (I) or formula (II) can be prepared by neutralizing a compound of the general formula (I) or formula (II) with an alkali metal hydroxide.

Desirably, the invention includes a compound of formula (I), where R¹ = R² = CH₃ and R³ = H, i.e. the compound of general formula (I) is N,N-dimethyl glycine (DMG).

Desirably, the invention includes a compound of formula (II), where R¹ = R² = R³ = H, i.e. the compound of general formula (I) is β-alanine (AL).

The alkali metal is not particularly limited. It can include sodium or potassium, but typically will be sodium.

The term "alkali metal" means the cation, e.g. the term "sodium" means sodium cation.

Desirably, the compounds corresponding to formulas I and II include Na-DMG and Na-AL, respectively.

The mixture employed in any of the methods of the invention used as a feed can comprise said alkali metal hydroxide in a quantity of from 0.05 to 5 % by weight, or from 0.1 to 5 % by weight, or from 0.5 to 5 % by weight, or from 1.0 to 5 % by weight, based on the weight of the mixture.

The mixture employed in any of the methods of the invention used as a feed can comprise said alkali metal salt of said compound of formula (I) desirably in a quantity of from 5 to 45 % by weight, based on the weight of the mixture. The mixture used as a feed can also comprise said alkali metal salt of said compound of formula (I) or formula (II) in a quantity of from 6 to 44 % by weight, or 7 to 43 % by weight, or from 10 to 40 % by weight.

Desirably, the mixture employed in any of the methods of the invention used as a feed can comprise a molar ratio of alkali metal hydroxide to the cumulative quantity of alkali metal salt of compounds corresponding to formulas I and II of at least 0.12, or at least 0.14, or at least 0.16, or at least 0.18, or at least 0.2.

Desirably, the molar ratio of the alkali metal hydroxide to the cumulative quantity of alkali metal salt of compounds corresponding to formulas I and II in the product composition according to the invention or the product composition obtained according to the methods of the invention can be no more than 0.02, or no more than 0.012, or no more than 0.014, or no more than 0.016, or no more than 0.018.

Desirably, the composition as defined in the claims comprising an alkali metal salt of the compound of formula (I) or (II) or the product composition obtained according to the methods of the invention as defined in the claims have to be regarded as a final product and not as an intermediate. Accordingly, it is desired that in the methods according to the invention besides the provision of a mixture comprising said salt, an alkali metal hydroxide and water, step (X) or step (X) and step (Y) are performed as the only method steps in terms of stabilizing or purifying an alkali metal salt of a compound of general formula (I) or (II). Thus, desirably, further method steps of stabilizing or purifying an alkali metal salt of a compound of formula (I) or (II) subsequent to steps (X) or steps (X) and (Y) are excluded.

In the following disclosure, the method according to the invention is mostly explained with reference to a mixture comprising Na-DMG, sodium hydroxide and water. However, it has to be understood that the method according to the invention is not limited to such specific mixture, but can be applied to a respective mixture containing an alkali metal salt of a compound of general formula (I) or formula (II), alkali metal hydroxide and water as defined in claim 1 or claim 2 as appended to this disclosure.

The inventors of the present invention as defined in the claims discovered that electrodialysis may be used for selectively reducing the quantity of sodium hydroxide in a stream or feed or feedstock which contains amino acids or amphoteric compounds. According to the invention, reduction of the quantity of alkali metal hydroxide in a mixture comprising an alkali metal salt of a compound of the general formula (I), alkali metal hydroxide and water by electrodialysis can be accomplished in the following non-limiting five embodiments (A1) to (A5). These methods may also be applied for reducing the quantity of alkali metal hydroxide in a mixture comprising an alkali metal salt of a compound of the general formula (II), alkali metal hydroxide and water.

The term "reducing the quantity of alkali metal hydroxide" or "reducing" or "reduction" as used herein encompasses the partial or complete removal of alkali metal hydroxide from a mixture comprising an alkali metal salt of a compound of the general formula (I) or (II), alkali metal hydroxide and water

Any of the processes of the invention can be conducted in a continuous or semi-continuous process.

Technique (A): Reducing the quantity of said alkali metal hydroxide by electrodialysis.

(A1) Direct reduction of the quantity of alkali metal hydroxide in the mixture by electrodialysis using a repeating unit with one anion-selective membrane (AM) and a cation-selective membrane (CM).

According to the first embodiment (A1) of technique (A), the quantity of alkali metal hydroxide is directly reduced in a mixture comprising an alkali metal salt of the compound of formula (I) or formula (II), alkali metal hydroxide and water, by using electrodialysis.

The term "directy reduced" means that alkali metal hydroxide is not subjected to a neutralization step with an acid prior to electrodialysis, but is reduced as such, i.e. as alkali metal hydroxide.

The term "electrodialysis (ED)" encompasses a method which is used to transport ions from one solution through ion-exchange membranes to another solution under the influence of an applied electric potential difference. In its broadest meaning, the cell comprises or consists of a feed (diluate) compartment and a concentrate compartment formed by suitable membranes placed between an anode and a cathode. The process of performing electrodialysis and suitable electrodialysis cells are known in the art.

In the method according to the first embodiment (A1) of technique (A), electrodialysis using anionic membranes AM and cationic membranes CM can be used to selectively reduce the quantity of alkali metal hydroxide in a feed which comprises an aqueous solution of the alkali metal salt of the compound according to formula (I), an alkali metal hydroxide, and optionally side-products.

In the following, the term "feed" is simultaneously used with the term "stream" or "feed stream".

The term "cationic membrane" is synonymously used with the term "cationic exchange membrane" or "cation-selective membrane", and the term "anionic membrane" is synonymously used with the term "anionic exchange membrane" or "anion-selective membrane".

**Fig. 1** shows an electrodialysis cell comprising two cationic membranes CM which are separated by an anionic exchange membrane AM in which the repeating unit is an anionic membrane and a cationic membrane. Thus, the arrangement CM/AM/CM forms two compartments, i.e. one compartment between CM and AM, and the other compartment between AM and CM.

The diluate feed stream comprises an aqueous solution of an alkali metal salt of the compound of general formula (I) or formula (II) comprising sodium hydroxide, which is fed to the first compartment formed by one of the two CMs and the AM. A concentrate feed comprising sodium hydroxide (MOH) is fed to the second compartment formed by the other one of the two CMs and the AM. Both feeds are allowed to flow through the appropriate cell compartments formed by the membranes. Under the influence of an electrical potential between the anode and the cathode, the anionic membrane AM is positively charged, whereas the cationic membrane CM is negatively charged.

Further under the influence of the electrical potential difference, the sodium ions (M⁺) in the feed of the aqueous alkali metal salt of the compound of general formula (I) or formula (II) migrate towards the negatively charged cathode and pass through the negatively charged cation exchange membrane CM. These sodium cations then stay in the concentrate, prevented from further migration towards the cathode by the positively charged anionic membrane AM. Hydroxide ions from the feed migrate through the AM into the concentrate compartment, which are prevented from further migration towards the anode by the negatively charged CM. Hydroxide ions in the feed compartment may migrate through the AM towards the anode. As a result of the anion and cation migration, electric current flows between the cathode and anode. Only an equal number of anion and cation charge equivalents are transferred from the feed stream into the concentrate stream and so the charge balance is maintained in each stream. The overall result of the electrodialysis process is a sodium ion concentration increase in the concentrate stream prevailing in the concentrate compartment with a depletion of sodium ions in the diluate solution feed stream prevailing in the feed compartment, and a hydroxide increase in the concentrate stream prevailing in the concentrate compartment with a depletion of hydroxide ions in the diluate stream prevailing in the diluate compartment. As a consequence, a composition comprising an alkali metal salt of formula (I) or formula (II) which is depleted with respect to sodium hydroxide is removed from the feed compartment, and a sodium hydroxide stream is removed from the concentrate compartment.

In another embodiment of the electrodialysis cell described above with respect to **Fig. 1**, the cell may comprise two anionic membranes which are separated by a cationic membrane.

(A2) Direct reduction of the quantity of the alkali metal hydroxide in the mixture by electrodialysis using a repeating unit which consists of a bipolar membrane (BM) and a cationic membrane (CM).

Electrodialysis using bipolar membranes (BM) can be used to selectively remove sodium hydroxide from a feed which contains an aqueous solution of the sodium salt of the compound according to general formula (I) or formula (II) comprising an alkali metal hydroxide and optionally side-products. The use of bipolar membranes makes it possible to create in situ protons that can exchange an alkali metal ion from the feed stream. At the same time, an alkali metal ion recombines with a hydroxide ion, the hydroxide ion also being produced by the bipolar membrane.

Thus, the electrodialysis cell can comprise two bipolar membranes BM which are separated by a cationic exchange membrane CM as indicated in **Fig. 2****,** and in which the repeating unit is a bipolar membrane and a cationic membrane. Thus, the arrangement BM/CM/BM forms two compartments.

The diluate feed stream comprises an aqueous solution of an alkali metal salt of formula (I) or formula (II) comprising sodium hydroxide, which is fed to the first compartment formed by one of the two BMs together with the CM. A concentrate feed comprising sodium hydroxide (MOH) is fed to the second compartment formed by the other one of the two BMs together with the CM. Both feeds are allowed to flow through the appropriate cell compartments. Under the influence of an electrical potential difference, the alkali metal ions (M⁺) in the feed of the aqueous an alkali metal salt of the compound of general formula (I) or formula (II) migrate towards the negatively charged cathode and pass through the negatively charged cation exchange membrane CM. These cations stay in the concentrate compartment, prevented from further migration towards the cathode by the bipolar membrane BM since the surface of it facing the CM is positively charged. Simultaneously, protons are produced at the surface of the bipolar membrane of the feed compartment facing the CM due to the ability of the BM to dissociate water into protons (and hydroxide ions which are formed at the surface of the BM of the concentrate compartment facing the anode, and which migrate towards the anode). These formed protons neutralize the hydroxide ions stemming from the sodium hydroxide being contained in the aqueous solution of the feed. Further simultaneously, hydroxide ions are produced at the surface of the BM of the concentrate compartment facing the CM (and protons which are formed at the surface of the BM facing the cathode, and which migrate towards the cathode). The hydroxide ions formed in the concentrate compartment are prevented from further migration towards the anode by the negatively charged CM. As a result of the anion and cation migration, electric current flows between the cathode and anode. Only an equal number of anion and cation charge equivalents are transferred from the feed stream into the concentrate stream and so the charge balance is maintained in each stream. The overall result of the electrodialysis process by this method is a hydroxide ion concentration increase in the concentrate stream in the concentrate compartment with a depletion of hydroxide ions in the diluate feed stream (since said hydroxide ions are neutralized by protons) in the diluate compartment, and a sodium ion increase in the concentrate stream with a depletion of sodium ions in the diluate stream. As a consequence, a composition comprising an alkali metal salt of the compound of general formula (I) or formula (II) which is depleted with respect to the quantity of alkali metal hydroxide and enriched in concentration of the acid of formula (I) or formula (II) is removed from the feed compartment, and sodium hydroxide (MOH) is removed from the concentrate compartment.

(A3) Reducing the quantity of the alkali metal hydroxide by neutralization with an acid and subsequent electrodialysis.

According to a third embodiment (A3) of technique (A), an acid (HX) is added to the mixture comprising an alkali metal salt of the compound of general formula (I) or formula (II), alkali metal hydroxide and water, i.e. the alkali metal hydroxide contained in said mixture is neutralized. Subsequent to the neutralization, electrodialysis is performed.

For neutralization of the alkali metal hydroxide contained in the mixture comprising said alkali metal salt of the compound of the general formula (I) or formula (II), said alkali metal hydroxide and water and optionally side-products, an inorganic acid can be used. Suitable acids are acids such as inorganic acids such as hydrochloric acid (HCI) and sulphuric acid (H₂SO₄).

After neutralization, wherein a salt is formed, desalination is performed using electrodialysis.

**Fig. 3** discloses an electrodialysis cell comprising a repeating unit which consists of an anionic exchange membrane AM and a cationic exchange membrane CM. Thus, the arrangement CM/AM/CM forms two com partments.

The diluate feed stream comprises an aqueous solution of an alkali metal salt of the compound of general formula (I) or formula (II) comprising sodium hydroxide, which has been neutralized with HCI (HX), i.e. which then comprises sodium chloride, and which is fed to the first compartment formed by one of the two CMs together with the AM. A concentrate feed comprising sodium chloride (MX) is fed to the second compartment formed by the other one of the two CMs and the AM. Both feeds are allowed to flow through the appropriate cell compartments. Under the influence of an electrical potential difference, the alkali metal ions (M⁺) in the feed of the aqueous solution of an alkali metal salt of the compound of general formula (I) or formula (II) migrate towards the negatively charged cathode and pass through the negatively charged cation exchange membrane CM. These cations stay in the concentrate compartment, prevented from further migration towards the cathode by the anionic membrane AM which is positively charged. Inorganic acid anions may migrate from the negatively charged cathode through the AM towards the positively charged anode. However, these inorganic acid ions stay in the concentrate compartment, prevented from further migration towards the anode by the cationic membrane (CM) which is negatively charged. On the other hand, chloride ions (X⁻) in the diluate may migrate towards the anode through the anionic membrane AM which is positively charged.

As a result of the anion (inorganic acid anions) and cation (alkali metal ions) migration, electric current flows between the cathode and anode. Only an equal number of anion and cation charge equivalents are transferred from the feed stream into the concentrate stream and so the charge balance is maintained in each stream. The overall result of the electrodialysis process is a concentration increase of sodium cations in the concentrate compartment/stream with a depletion of sodium ions in the diluate compartment/feed stream. As a consequence, a composition comprising an alkali metal salt of the compound of general formula (I) or formula (II) which is depleted with respect to sodium chloride is removed from the feed compartment, and sodium chloride (MX) is removed from the concentrate compartment.

(A4) Reducing the quantity of alkali metal hydroxide by neutralization of the alkali metal hydroxide with an acid and simultaneous electrodialysis.

In this embodiment (A4) of technique (A), alkali metal ions (M⁺) are replaced by protons which are added to the electrolysis cell. The solution that is used to provide the protons for neutralizing the alkali metal hydroxide in said mixture is also the solution that receives the alkali metal ions that are removed out of the product.

**Fig. 4** discloses an electrodialysis cell comprising a repeating unit which consists of two cationic membranes CM. Thus, the arrangement CM/CM/CM forms two compartments.

A stream of acid HX, e.g. HCI, is fed to the first compartment formed by two adjacent CMs. The diluate feed stream comprises an aqueous solution of an alkali metal salt of the compound of general formula (I) or formula (II) comprising sodium hydroxide which is fed to the second compartment formed by the other two adjacent CMs. Both feeds are allowed to flow through the appropriate cell compartments formed by the CMs. Under the influence of an electrical potential difference, protons from the acid stream (HX) migrate towards the negatively charged cathode and pass through the negatively charged cation exchange membrane CM into the compartment containing the diluate feed comprising an alkali metal salt of the compound of general formula (I) or formula (II) and sodium hydroxide. Accordingly, alkali metal hydroxide contained in the feed is neutralized. The alkali metal ions (M⁺) in the feed of the aqueous alkali metal salt of the compound of general formula (I) or formula (II) migrate towards the negatively charged cathode and pass through the negatively charged cation exchange membrane (CM). Further sodium ions may migrate from the anode through the CM into the concentrate compartment, which are prevented from further migration into the diluate compartment by the negatively charged further CM.

As a result of the cation (alkali metal ions and H⁺) migration, electric current flows between the cathode and anode. Only an equal number of anion and cation charge equivalents are transferred from the feed stream into the concentrate stream and so the charge balance is maintained in each stream. The overall result of the electrodialysis process is a concentration increase of sodium cations in the concentrate compartment/stream with a depletion of alkali metal ions in the diluate solution compartment/feed stream. As a consequence, a composition comprising an alkali metal salt of the compound of general formula (I) or formula (II) which is depleted with respect to alkali metal hydroxide is removed from the feed compartment, and the metal salt of the inorganic acid together with the inorganic acid is removed from the concentrate compartment.

(A5) Reducing the quantity of alkali metal hydroxide by neutralization of the alkali metal hydroxide with an acid and simultaneous electrodialysis.

In this embodiment (A5) of technique (A), alkali metal ions are replaced by protons which are added to the electrolysis cell.

**Fig. 5** discloses an electrodialysis cell comprising a repeating unit which consists of one anionic membrane AM and two cationic exchange membranes CM. Thus, the arrangement AM/CM/CM/AM forms three compartments.

The diluate feed stream fed to the compartment formed by the two CMs comprises an aqueous solution of an alkali metal salt of the compound of general formula (I) or formula (II) comprising alkali metal hydroxide. An acid HX, e.g. HCI, is fed to the compartment formed by one of the two AMs and one of the two CMs. A concentrate feed comprising chloride metal salt of an inorganic acid (MX) is fed to the compartment formed by the other one of the two AMs and the other one of the two CMs. Both feeds are allowed to flow through the appropriate cell compartments. Under the influence of an electrical potential difference, protons from the inorganic acid stream may migrate through the CM into the feed compartment, thereby neutralizing hydroxide ions prevailing there. The alkali metal ions in the feed of the aqueous alkali metal salt of the compound of general formula (I) or formula (II) migrate towards the negatively charged cathode and pass through the negatively charged cation exchange membrane CM. These cations stay in the concentrate compartment/stream, prevented from further migration towards the cathode by the anionic membrane AM which is positively charged. inorganic acid anions may migrate from the negatively charged cathode through the AM towards the positively charged anode. However, these inorganic acid anions stay in the concentrate, prevented from further migration towards the anode by the cationic membrane CM which is negatively charged. On the other hand, chloride ions in the acid feed may migrate towards the anode through the anionic membrane which is positively charged.

As a result of the anion (e.g. Cl⁻) and cation (H⁺, alkali metal salts) migration, electric current flows between the cathode and anode. Only an equal number of anion and cation charge equivalents are transferred from or to each stream and so the charge balance is maintained in each stream. The overall result of the electrodialysis process is a concentration increase of sodium cations in the concentrate stream with a depletion of alkali metal ions in the diluate solution feed stream, and a depletion of hydroxide ions in the diluate feed and an increase of chloride ions in the concentrate. As a consequence, a composition comprising an alkali metal salt of the compound of general formula (I) or formula (II) which is depleted with respect to alkali metal hydroxide is removed from the feed compartment, and alkali metal of the inorganic acid is removed from the concentrate compartment. The inorganic acid is removed from the acid compartment.

By applying a balanced residence time and current density, selective removal of the sodium hydroxide in a feed of an alkali metal salt of the compound of general formula (I) or formula (II) was shown with limited transport of a compound of formula (I) or formula (II) through the membrane and with limited neutralization of the alkali metal salt of the compound of general formula (I) or formula (II) into the free acid.

In a further embodiment (not shown in the figures), the electrodialysis cell comprises a repeating unit which consists of two adjacent anion-selective membranes AM and one cation-selective membrane CM. Thus, the arrangement AM/AM/CM/AM forms three compartments. Said alkali metal hydroxide contained in said mixture is neutralized by addition of an acid simultaneously to subjecting said mixture to electrodialysis.

In the purification methods according to embodiments (A1) and (A2), alkali metal hydroxide is advantageously formed which can be used in processes of preparing an alkali metal salt of a compound of the general formula (I) or formula (II) as disclosed in the Background section. This may be an advantage under economic aspects.

Contrary to this, the purification methods according to embodiments (A3) to (A5) consume an inorganic acid and/or produce alkali metal of the used inorganic acid.

Accordingly, purification methods according to embodiments (A1) and (A2) may be desirable over purification methods according to embodiments (A3) to (A5).

Furthermore, embodiment (A2) has the advantage of forming protons, which can be used for neutralization. Therefore, the method has the benefit of complete or nearly complete and fast selective reduction of alkali metal ions and hydroxide ions from a mixture comprising an alkali metal salt of a compound of general formula (I) or formula (II), alkali metal hydroxide and water. Furthermore, the migration of organic compounds is advantageously limited.

Summing up, in technique (A), said electrodialysis may be performed in an electrodialysis cell comprising at least one anionic membrane; or one cationic membrane; or one bipolar membrane; or one anionic membrane and one cationic membrane; or one cationic membrane and one bipolar membrane; or one anionic membrane and one bipolar membrane; or one anionic membrane, one cationic membrane and one bipolar membrane.

In any of the (A) methods, said electrodialysis cell can comprise at least one bipolar membrane.

In any of the (A) methods, said electrodialysis cell can comprise at least one bipolar membrane and one cationic membrane.

In any of the methods of technique (A), said electrodialysis can be performed in an electrodialysis cell:
comprising two anionic membranes which are separated from one another by a cationic membrane, thus forming a two-compartment arrangement; or
comprising two cationic membranes, which are separated from one another by a cationic membrane, thus forming a two-compartment arrangement; or
comprising two bipolar membranes which are separated from one another by a cationic membrane, thus forming a two-compartment arrangement; or
comprising two anionic membranes which are separated from one another by a cationic membrane, thus forming a two-compartment arrangement, and wherein said alkali metal hydroxide comprised in said mixture is neutralized by addition of an acid prior to subjecting said mixture to electrodialysis; or
comprising two cationic membranes which are separated from one another by an anionic membrane, thus forming a two-compartment arrangement, and wherein said alkali metal hydroxide comprised in said mixture is neutralized by addition of an acid simultaneously to subjecting said mixture to electrodialysis; or
comprising two cationic membranes which are separated from one another by a further cationic membrane, thus forming a two-compartment arrangement, and wherein said alkali metal hydroxide comprised in said mixture is neutralized by addition of an acid simultaneously to subjecting said mixture to electrodialysis; or
comprising two anionic membranes which are separated from one another by two cationic membrane, thus forming a three-compartment arrangement, and wherein said alkali metal hydroxide comprised in said mixture is neutralized by addition of an acid simultaneously to subjecting said mixture to electrodialysis.

Desirably, said electrodialysis is performed in an electrodialysis cell comprising two bipolar membranes which are separated from one another by a cationic membrane, thus forming a two-compartment arrangement.

Any of the methods of technique (A) may be run in batch, feed-and-bleed system and continuous system. The term "feed-and-bleed system" encompasses a system by means of which it is possible to achieve relative constant concentrations of the individual substances in a mixture or bath during production over a prolonged period. Degradation products formed during the production process to be diluted periodically in this way can be maintained for a prolonged period below a critical concentration.

In one embodiment, multiple electrodialysis cells are arranged into a configuration called an electrodialysis stack.

In any of the methods of the invention for reducing the quantity of alkali metal hydroxide [whether method (A) or method (B)], the feed (or mixture) to be treated (whether by electrodialysis or neutralization or any other method) contains an alkali metal salt of the compound of general formula (I) or formula (II) in an quantity of from 0.1 wt% and up to 50 wt%, or from 5 wt.% and up to 50 wt.%, or from 10 wt.% and up to 50 wt.%, or from 5 wt.% and up to 45 wt%, or from 5 wt.% and up to 40 wt%, or from 10 to 30 wt%, or from 15 to 25 wt%, or from 20 to 25 wt%, in each case based on the weight of the feed or mixture. Along with the aforementioned quantity of alkali metal salt, or independently, the feed or mixture contains from 0.1 and up to 5 wt% of an alkali metal hydroxide, based on the weight of the feed (or mixture). The feed or the mixture also contains water.

The composition according to the invention is defined in the claims. Also disclosed is a product stream or a composition comprising an alkali metal salt of a compound of the general formula (I) or formula (II)

R¹R²N-CHR³-COOH (I)

or

R¹R²N-CHR³CHR³-COOH (II),

wherein R¹, R² and R³ are independently H, an alkyl, an alkenyl, or a hydroxyalkyl radical containing from 1 to 18 carbon atoms, or from 1 to 6 carbon atoms; or wherein R¹ and R² form jointly together with the N atom a heterocyclic 5- or 6-membered ring;
and an alkali metal hydroxide in a quantity of less than 0.5, or not more than 0.1 % by weight, based on the weight of the alkali metal salt of the compound of the general formula (I) or formula (II) and, if any is present, the alkali metal hydroxide; and
a chloride content of not more than 75 ppm and a sulfate content of not more than 750 ppm, based on the total weight of the composition.

The amount of alkali metal hydroxide in the composition or product stream is desirably not more than 0.1 wt%, or not more than 0.05 % by weight, or not more than 0.025 wt.%, or not more than 0.015 wt.%, or not more than 0.01 wt.%, or not more than 0.005 wt.%, based on the weight of the alkali metal salt of the compound of formula (I) or (II) and alkali metal hydroxide.

The amount of chloride in the composition or product stream, if any, is desirably not more than 75 ppm, or not more than 50 ppm, or not more than 30 ppm, or not more than 20 ppm, or not more than 10 ppm, or not more than 5 ppm, and can be absent of any detectable chloride (e.g. 0 ppm or below a detectable limit set to 0.5 ppm which is deemed equivalent to 0 ppm).

The amount of sulfate in the composition or product stream, if any, is desirably not more than 750 ppm, or not more than 500 ppm, or not more than 300 ppm, or not more than 100 ppm, or not more than 50 ppm, or not more than 10 ppm, and can be absent of any detectable sulfate (e.g. 0 ppm or below a detectable limit set to 0.5 ppm which is deemed equivalent to 0 ppm).

Optionally, the above composition or product stream may contain N-methylglycine, or sarcosine, present in an amount of at least 0.1 wt. %, or at least 0.15 wt.%, and in each case up to about 0.3 wt.%, or up to 0.2 wt.%, based on the weight of the composition, or product stream. The presence of sarcosine can be due to some dealkylation of dimethylglycine on the catalysts used in a DMAE process. The above composition or product streams can be obtained by any of the methods described herein.

In any one or more of the methods described herein, the percent reduction in the quantity of alkali metal hydroxide in the product stream obtained in electrolysis relative to the feed is desirably at least 70 %, or at least 80 %, or at least 90 %, or at least 95 %, or at least 98 %, or at least 99 %, relative to the quantity of alkali metal hydroxide in the feed (or mixture).

Ion exchange membranes suitable for performing the method according to the invention according to technique (A) are known and are commercially available. Cationic membranes (CM) are e.g. available under the trademark SK^{®} membranes from PCA. Anionic membranes (AM) are e.g. available under the trademark Neosepta^{®} AHA from Eurodia and Fumasep^{®} FAA-3-PK-130 from Fumatech. Bipolar membranes (BM) are e.g. available under the trademarks PCA BPM^{®}, Neosepta^{®} BP-1 and Fumasep^{®} FBM.

Despite the fact that the amino acid salts are also present in the mixture in their ionic form, the inventors have surprisingly found that the removal of alkali metal hydroxide such as NaOH from the feed across the ED membranes could occur with excellent selectivity. Little to no amino acids were lost in the concentrate streams.

(B) Reducing the quantity of alkali metal hydroxide by neutralization of the alkali metal hydroxide by addition of an acid.

According to the second technique (B), the quantity of said alkali metal hydroxide contained in a mixture comprising said salt, said alkali metal hydroxide and water, can be reduced by adding an acid, i.e. the quantity may be reduced by neutralization. Accordingly, an acid is added to the mixture in a quantity sufficient to neutralize at least a portion, or at least 95 %, or all of said alkali metal hydroxide.

The acid employed for neutralization can be an inorganic acid, an organic acid, or a mixture thereof.

In one embodiment, an inorganic acid is used for neutralization. Any inorganic acid may be used, provided said acid does not negatively affect the intended use, i.e. the use of the purified salt of a compound of the general formula (I) or formula (II) as a feed additive. Examples of suitable inorganic acids are hydrochloric acid, sulfuric acid, and phosphoric acid.

In another embodiment, an organic acid is used for neutralization. Any organic acid may be used, provided said acid does not negatively affect the intended use, i.e. the use of the purified salt of a compound of the general formula (I) or formula (II) as a feed additive. Examples of suitable organic acids are acetic acid, benzoic acid, or lactic acid. Amino acids may also be used.

Desirably, said organic acid can be a compound of the general formula (I), i.e. R¹R²N-CHR³-COOH, or a compound of the general formula (II), i.e. R¹R²N-CHR³CHR³-COOH, wherein each R group can be selected as described with reference to formula (I) or formula (II) of the alkali metal salt, or can be the free acid version of the same formula as the alkali metal salt.

If desired, said alkali metal hydroxide contained in the mixture at least comprising said salt, said alkali metal hydroxide and water, can be reduced indirectly by ion exchange.

The term "reduced indirectly" means that desirably alkali metal hydroxide is subjected to a neutralization step with an acid corresponding to formula (I) or formula (II) which is generated by treating the corresponding alkali metal salt of the compound of formula (I) or formula (II) by an ion exchange technique.

Accordingly, in the indirect reduction method of (B), a portion of a starting mixture comprising an alkali metal salt of a compound of the general formula (I) or (II), alkali metal hydroxide, and water, is withdrawn as an ion exchange feed composition comprising the alkali metal salt of the compound of the general formula (I) or (II), alkali metal hydroxide, and water, and the remaining portion of the starting mixture is an untreated mixture. The ion exchange feed composition is brought into contact with an acidic ion exchanger, resulting in a treated ion exchange composition. In the acidic ion exchanger, alkali metals ions, i.e. both from the alkali metal hydroxide and from the salt of the compound of formula (I) or formula (II), are replaced by protons resulting in the formation of an acid of formula (I) or (II), e.g. (I) R¹R²N-CHR³-COOH or (II) R¹R²N-CH₂CH₂-COOH, and water, to produce an ion exchange treated composition. After mixing the ion exchange treated composition with the untreated mixture, alkali metal hydroxide present in the untreated mixture is neutralized by the newly formed acid contained in the ion exchange treated composition of formula (I) or (II), e.g. R¹R²N-CHR³-COOH or R¹R²N-CH₂CH₂-COOH, and water. Thus, the quantity of alkali metal hydroxide has been reduced.

Accordingly, said acid compound of formula (I) or formula (II) which is added in technique (B) to said mixture is prepared by subjecting a part of said mixture comprising said alkali metal salt of the compound of formula (I) or formula (II) to ion exchange using an acidic ion exchanger.

**Fig. 6** schematically shows a process of removing or at least reducing the quantity of alkali metal hydroxide from an aqueous mixture of an alkali metal salt of a compound of general formula (I) or formula (II), optionally alkyl amino alcohols (AAA) being contained in the mixture, and alkali metal hydroxide. The alkali metal salt of formula (I) or formula (II) has been prepared in a reactor according to the third route as defined in the Background section.

In the ion exchange system (lonex), the crude mixture of the alkali metal salt of the compound of general formula (I) or formula (II) together with the alkali metal hydroxide is passed through an acidic exchange resin (Ionex) to reduce substantially all of the sodium ions. Desirably, two or more ion exchange resins columns are arranged in parallel, so that one column, or several columns in series, is used to reduce the quantity of sodium ions while the others are being regenerated. The regeneration of exhausted ion exchange resins is conducted using an acidified regenerant. In the preferred process, the regenerant is recycled and re-used in a following regeneration procedure. R¹R²N-CHR³-COOH or R¹R²N-CH₂CH₂-COOH obtained by ion exchange is then added to the untreated stream having not been subjected to ion exchange according to technique (B).

In the ion exchange system, the ion exchange feed composition to the ion exchange may contain an alkali metal salt of the compound of general formula (I) or formula (II) in an quantity from 0.1 wt.% and up to 50 wt.%, or from 5 wt. % and up to 50 wt. %, or from 10 wt. % and up to 50 wt. %, or from 5 wt. % and up to 45 wt. %, or from 5 wt. % and up to 40 wt. %, or from 10 to 30 wt.%, or from 15 to 25 wt.%, or from 20 to 25 wt.%, in each case based on the weight of the ion exchange feed composition. Along with the aforementioned quantity of alkali metal salt, or independently, the ion exchange feed composition contains at least 0.1 wt.%, or from 0.1 and up to 5 wt.%, of an alkali metal hydroxide. The ion exchange feed composition also contains water and optionally small quantities of alkyl amino alcohols (AAA). After mixing both streams together, i.e. the untreated composition with the ion exchange treated composition, a product composition is obtained at the outlet of the system, and the product composition comprises less than 0.1 wt.%, or 0.05 wt.% or less, or 0.025 wt.% or less, or 0.01 wt.% or less, or 0.005 wt.% or less alkali metal hydroxide, based on the weight of the alkali metal salt of the compound of formula (I) or (II) and alkali metal hydroxide. The quantity of ion exchange feed withdrawn from the mixture is effective to reduce the alkali metal hydroxide content to the desired level with the particular kind of ion exchange composition employed, and these may be determined by routine experimentation.

The pH range of the mixture to be treated comprising the alkali metal salt of the compound of general formula (I) or formula (II) typically is between 10.5 and 11.5.

Suitable acidic ion exchangers can be of the weak or strong acid type, and can be either gel type or macroporous type. These materials are known and are commercially available. Suitable acidic ion exchangers are e.g. commercially available under the trademark Dowex (e.g. Dowex 88), Amberjet^{®} (e.g. Amberjet^{®} 1500H) or Amberlite^{®} 'e.g. Amberlite^{®} IRCE86) from The Dow Chemical Company, or Lewatit^{®} MonoPlus S 200 KR or Lewatit^{®} CNP 80 WS from Lanxess.

The sodium salt of compound of formula (II) can be treated in the same manner as the sodium salt of the compound of formula (I). Accordingly, a stream containing the sodium salt of β-alanine, NaOH, and water, is neutralized with a stream obtained after ion exchange of the sodium salt of β-alanine.

The combination of both techniques may result in sodium salts of the compounds of formula (I), respectively formula (II), containing a particularly low quantity of sodium hydroxide.

Subsequent to the removing of said alkali metal hydroxide according to techniques (A) or (B) or techniques (A) and (B), the resulting product may be concentrated and/or may be dried, if desired.

Accordingly, in one embodiment, the method further comprises step (Y):
(Y) concentrating or concentrating and drying the product obtained according to the claimed methods.

In a second aspect, the invention relates to a composition as defined in the claims. Also disclosed is a composition comprising an alkali metal salt of a compound of the general formula (I) or formula (II)

R¹R²N-CHR³-COOH or R¹R²N-CHR³CHR³-COOH (I) (II),

wherein R¹, R² and R³ are independently H, an alkyl, an alkenyl, or a hydroxyalkyl radical containing from 1 to 18 carbon atoms, or from 1 to 6 carbon atoms; or wherein R¹ and R² form jointly together with the N atom a heterocyclic 5- or 6-membered ring;
and an alkali metal hydroxide in a quantity of less than 0.5 % by weight in said composition, or less than 0.1 % by weight, based on the weight of the alkali metal salt of the compound of the general formula (I) or formula (II) and alkali metal hydroxide.

In one embodiment, the composition is obtainable by a method as defined in the first aspect.

Desirably, the molar ratio of the hydroxide to alkali metal salt of compounds corresponding either of formulas I or II in the product composition according to the invention or obtained according to the methods of the invention can be no more than 0.012, or 0.014, or 0.016, or 0.018, or 0.02.

In a third aspect, the invention relates to the use of a composition as defined in the second aspect or obtained according to the method defined in the first aspect as feed additive.

In one embodiment, said feed additive is for poultry, with the alkali metal salt of compound of formula (I).

In another embodiment, said feed additive is for pig, with the alkali metal salt of compound of formula (II).

### EXAMPLES

### Example 1: Electrodialysis according to embodiment (A2):

A mixture comprising Na-DMG (19.0 wt.%), DMAE (3.1 wt.%), NaOH (0.5 wt.%) and water was subjected to electrodialysis using a repeating unit which consists of a bipolar membrane (BM) and a cationic membrane (CM).

The electrodialysis stack contained 5 membrane cell pairs with an active surface area of 64 cm² for each membrane. Fumasep^{®} FBM membranes were used as bipolar membrane (BM) and Fumasep^{®} FKB membranes as cation membrane (CM). Membrane spacer thickness was 0.75 mm. The stack voltage drop was 13.5 V. The recirculation flow rate was 60 L/h for the diluate and concentrate compartments and 150 L/h for the electrode rinse compartment. An 85.0 g/L Na₂SO₄ solution was used as electrode rinse solution in a closed loop set-up. For each compartment, the pressure drop was kept below 0.5 bar.

The electrodialysis experiment was performed in feed-and-bleed mode. Once the power source was switched on, the experiment was run for 1000 hours. To the diluate compartment, fresh Na-DMG mixture was fed. The diluate feed flow rate was increased continually from 1.6 kg/h to 2.5 kg/h during the experiment. To the concentrate compartment, demineralized water was fed initially. The concentrate feed flow rate ranged from 220-250 g/h.

During the experiment, complete NaOH removal from the Na-DMG mixture was achieved. Moreover, an excess quantity of Na bound to DMG was removed from the mixture, producing varying quantities of H-DMG. The produced quantity of H-DMG in the diluate ranged from 2.8 wt% to 0.3 wt% as the diluate residence time was lowered. The concentration of NaOH in the outlet of the concentrate compartment was 7 wt%. For DMG-Na, loss to the concentrate compartment stayed below 0.6 % and below 0.9 % for DMAE. These losses are expressed relative to the total quantity of the respective compounds fed to the diluate compartment.

This experiment proved that complete removal of NaOH is possible from a mixture comprising Na-DMG, DMAE, NaOH and water by electrodialysis with bipolar membranes with minimal losses of the principal compounds to the concentrate compartment. Moreover, a 7 % NaOH stream is generated during this experiment, that is useful in chemical processes, for instance as recycle to the Na-DMG manufacturing process.

### Example 2: Electrodialysis according to embodiment (A3)

A mixture comprising Na-DMG (18.0 wt.%), DMAE (3.0 wt.%), NaOH (0.9 wt.%) and water was subjected to electrodialysis as diluate. Prior to the experiment, all NaOH was neutralized by adding the appropriate quantity of 37 wt% HCI (aq.) to the diluate. This resulted in an initial diluate chloride concentration of 51 g/L and a pH of 9. As a feed to the concentrate compartment, a 40 g/L NaCl (aq.) solution was used at start-up.

The electrodialysis stack contained 5 membrane cell pairs with an active surface area of 64 cm² for each membrane. PCA^{®} MVA membranes were used as anion membrane and PCA^{®} MVK membranes as cation membrane. Membrane spacer thickness was 0.75 mm. The stack voltage drop was 7.5 V. The recirculation flow rate was 30 L/h for the diluate and concentrate compartments and 150 L/h for the electrode rinse compartment. An 85.0 g/L Na₂SO₄ solution was used as electrode rinse solution. For each compartment, the pressure drop was kept below 0.5 bar. The initial volume was 2 L for both the diluate and concentrate compartments.

Electrodialysis was performed in batch mode. Once the power source was switched on, the experiment was run for five hours.

At the end of the experiment, the remaining diluate sodium and chloride concentrations were 0.2 g/L and 1.0 g/L respectively. A loss of 1.4 % for Na-DMG and 16 % for DMAE to the concentrate was observed. These percentages are relative to the total quantity of the respective compounds in the initial, neutralized diluate. The final concentration of NaCl in the concentrate was 100 g/L.

This experiment proved that complete removal of NaOH is possible from a mixture comprising Na-DMG, NaOH and water. NaOH removal is achieved through neutralization by HCI and subsequent removal of NaCl by electrodialysis. Loss of the principal compound, Na-DMG, is minimal and 2 % of the initial NaCl is retained in the diluate mixture.

### Example 3: Electrodialysis according to embodiment (A2)

A mixture comprising Na-β-alaninate (47.9 wt.%), NaOH and water was subjected to electrodialysis using a repeating unit which consists of a bipolar membranes (BM) and a cationic membrane (CM). The quantity of NaOH in the Na-β-alaninate mixture was 2.43 wt.%.

The electrodialysis stack contained 5 membrane cell pairs with an active surface area of 64 cm² for each membrane. Fumasep^{®} FBM membranes were used as bipolar membrane (BM) and Fumasep^{®} FKB membranes as cation membrane (CM). Membrane spacer thickness was 0.75 mm. The stack voltage drop was 13.5 V. The recirculation flow rate was 60 L/h for the diluate and concentrate compartments and 150 L/h for the electrode rinse compartment. An 85.0 g/L Na₂SO₄ solution was used as electrode rinse solution in a closed loop set-up. For each compartment, the pressure drop was kept below 0.5 bar.

Electrodialysis was performed in batch mode. Once the power source was switched on, the experiment was run for 1.5 hours.

During the experiment, complete NaOH removal from the β-alaninate mixture was achieved. Moreover, an excess quantity of Na bound to β-alaninate was reduced from the mixture, producing varying quantitys of β-alanine. The produced quantity of β-alanine in the diluate was 1.03 wt.%. The concentration of NaOH in the outlet of the concentrate compartment was 4 wt.%. For Na-β-alaninate, loss to the concentrate compartment stayed below 5 %. These losses are expressed relative to the total quantity of the respective compounds fed to the diluate compartment.

This experiment proved that complete removal of NaOH is possible from a mixture comprising Na-β-alaninate and water by electrodialysis with bipolar membranes with minimal losses of the principal compounds to the concentrate compartment. Moreover, a 4 % NaOH stream is generated during this experiment, that is useful in chemical processes, for instance as recycle to the Na-β-alaninate manufacturing process.

### Example 4: Reducing the quantity of sodium hydroxide by addition of a weak acid ion exchange resin

A mixture comprising Na-DMG (19.8 wt.%), DMAE (2.8 wt.%), NaOH (0.8 wt.%) and water was subjected to treatment with a weak acid ion exchange resin.

A double-walled column equipped with a preheater was filled with a 200 mL bed volume of Lewatit ^{®} CNP 80 WS weak acid resin. A constant feed flow rate of 2 L/h was used. The column was kept at a constant temperature of 40°C.

Column depletion was achieved after feeding 3,200 mL of the Na-DMG solution. During depletion, the Na-DMG mixture was collected. Subsequently, the column was rinsed with 1,200 mL of demineralized water. Resin regeneration was achieved by feeding 1,200 mL of 6 wt.% HCl (aq.) solution. Finally, the column was rinsed with 1,200 mL of RO-water (reverse osmosis water). This cycle was executed 100 times.

For all cycles, the collected Na-DMG mixture was sufficiently neutralized (i.e. complete NaOH removal) and had a pH value below 11.5. Resin capacity was found to remain constant during the whole of the experiment with a value of 3.9 eq Na⁺/L. No significant loss of the principal compounds in the Na-DMG mixture to the regenerate stream was observed.

This experiment proved that complete removal of NaOH is possible from a mixture comprising Na-DMG, NaOH and water. NaOH removal is achieved through treatment with a weak acid ion exchange resin. The capacity for Na⁺ removal is completely sustained after 100 depletionregeneration cycles. Moreover, no loss of the principal compounds in the Na-DMG mixture to the regenerate stream was observed.

### Example 5: Ion exchange experiment: Production of N,N-dimethylglycine

A certain part of 1,190 mL mixture comprising Na-DMG, DMAE, NaOH and water was subjected to treatment with a weak acid ion exchange resin. The quantity of NaOH in the Na-DMG mixture was 1.1 wt.%. Na-DMG and DMAE were present in a concentration of 17.2 wt.% and 3.0 wt.% respectively.

A double-walled column equipped with a preheater was filled with a 400 mL volume of Lewatit ^{®} CNP 80 WS weak acid resin. The space between the beads was filled with demineralized water. A part of the Na-DMG mixture was fed to the column at a constant flow rate of 4 L/h. The column was kept at a constant temperature of 40 °C.

During the experiment, pH of the column effluent was monitored. At start-up, a sudden drop of the effluent pH from 4 to 2 indicated the displacement of the dead volume of demineralized water. This fraction was collected as waste. Subsequently, 200 mL of mixture was collected at the bottom of the column, containing 0 wt.% NaOH, 14.0 wt.% DMG-H and 3.1 wt.% DMAE.

This mixture (200 mL) was recombined with the remaining 990 mL of the parent DMG-Na mixture which was not treated with a weak acid ion exchange resin. The resulting mixture was found to contain 0 wt.% NaOH, 16.6 wt.% DMG-Na and 3.0 wt.% DMAE.

## Claims

1. Method of stabilizing an alkali metal salt of a compound of the general formula (I) or formula (II)
R¹R²N-CHR³-COOH or R¹R²N-CHR³CHR³-COOH (I) (II),
wherein R¹, R² and R³ are independently H, an alkyl, an alkenyl, or a hydroxyalkyl radical containing from 1 to 18 carbon atoms, or from 1 to 6 carbon atoms; or wherein R¹ and R² form jointly together with the N atom a heterocyclic 5- or 6-membered ring;
said method comprising providing a mixture comprising said salt, an alkali metal hydroxide and water, and subjecting the mixture to a step (X):
(X) reducing the quantity of said alkali metal hydroxide in the mixture wherein step
(X) comprises
(A) reducing the quantity of said alkali metal hydroxide in the mixture by subjecting the mixture to electrodialysis; or
(B) reducing the quantity of said alkali metal hydroxide in the mixture by adding an acid to the mixture and (A) reducing the quantity of said alkali metal hydroxide in the mixture by subjecting the mixture to electrodialysis;
wherein in step (A) said electrodialysis is performed in an electrodialysis cell whose repeating unit consists of
an anionic membrane and a cationic membrane and two compartments; or
a bipolar membrane and a cationic membrane and two compartments; or
a bipolar membrane and an anionic membrane and two compartments; or
a bipolar membrane, an anionic membrane and a cationic membrane and three compartments; or
an anionic membrane and a cationic membrane and two compartments wherein said alkali metal hydroxide comprised in said mixture is neutralized by addition of an acid prior to subjecting said mixture to electrodialysis; or
two cationic membranes in a two compartment arrangement, and wherein said alkali metal hydroxide comprised in said mixture is neutralized by addition of an acid simultaneously to subjecting said mixture to electrodialysis; or
two adjacent cationic membranes, one anionic membrane and three compartments, and wherein said alkali metal hydroxide comprised in said mixture is neutralized by addition of an acid simultaneously to subjecting said mixture to electrodialysis; or
two adjacent anionic membranes, one cationic membrane and three compartments, and wherein said alkali metal hydroxide comprised in said mixture is neutralized by addition of an acid simultaneously to subjecting said mixture to electrodialysis;
wherein the acid added in step (B) is a compound of formula (I) or formula (II); or wherein the acid added in step (B) is obtained by subjecting at least a part of said mixture to ion exchange using an acidic ion exchanger.

2. Method of purifying an alkali metal salt of a compound of the general formula (I) or formula (II)
R¹R²N-CHR³-COOH or R¹R²N-CHR³CHR³-COOH (I) (II),
wherein R¹, R² and R³ are independently H, an alkyl, an alkenyl, or a hydroxyalkyl radical containing from 1 to 18 carbon atoms, or from 1 to 6 carbon atoms; or wherein R¹ and R²form jointly together with the N atom a heterocyclic 5- or 6-membered ring;
said method comprising providing a mixture comprising said salt, an alkali metal hydroxide and water, and subjecting the mixture to a step (X):
(X) reducing the quantity of said alkali metal hydroxide in the mixture;
wherein
(A) said alkali metal hydroxide is reduced by subjecting said mixture to electrodialysis; or
(B) said alkali metal hydroxide is reduced by adding an acid to said mixture and (A) said alkali metal hydroxide is reduced by subjecting said mixture to electrodialysis;
wherein in step (A) said electrodialysis is performed in an electrodialysis cell whose repeating unit consists of
an anionic membrane and a cationic membrane and two compartments; or
a bipolar membrane and a cationic membrane and two compartments; or
a bipolar membrane and an anionic membrane and two compartments; or
a bipolar membrane, an anionic membrane and a cationic membrane and three compartments; or
an anionic membrane and a cationic membrane and two compartments wherein said alkali metal hydroxide comprised in said mixture is neutralized by addition of an acid prior to subjecting said mixture to electrodialysis; or
two cationic membranes in a two compartment arrangement, and wherein said alkali metal hydroxide comprised in said mixture is neutralized by addition of an acid simultaneously to subjecting said mixture to electrodialysis; or
two adjacent cationic membranes, one anionic membrane and three compartments, and wherein said alkali metal hydroxide comprised in said mixture is neutralized by addition of an acid simultaneously to subjecting said mixture to electrodialysis; or
two adjacent anionic membranes, one cationic membrane and three compartments, and wherein said alkali metal hydroxide comprised in said mixture is neutralized by addition of an acid simultaneously to subjecting said mixture to electrodialysis;
wherein the acid added in step (B) is a compound of formula (I) or formula (II); or wherein the acid added in step (B) is obtained by subjecting at least a part of said mixture to ion exchange using an acidic ion exchanger.

3. Method of any one of the preceding claims, wherein said alkali metal salt of the compound of formula (I) or formula (II) is prepared in a process for making a compound of formula (I) or formula (II) in the presence of an alkali metal hydroxide; or wherein said alkali metal salt of the compound of formula (I) or formula (II) is prepared by contacting the compound of formula (I) or formula (II) with an alkali metal hydroxide.

4. Method of any one of the preceding claims, wherein
in compound of formula (I) R¹ = R² = CH₃ and R³ = H; or
in compound of formula (II) R¹ = R² = R³ = H.

5. Method of any one of the preceding claims, wherein said alkali metal comprises sodium or is sodium.

6. Method of any one of the preceding claims, wherein said mixture comprises said alkali metal hydroxide in a quantity of from 0.05 to 5 % by weight, based on the weight of the mixture; or wherein said mixture comprises said alkali metal salt of said compound of formula (I) or formula (II) in a quantity of from 5 to 45 wt.%, based on the weight of the mixture.

7. Method of any one of claims 1 to 6, wherein in step (A) said electrodialysis is performed in an electrodialysis cell comprising at least
one anionic membrane; or
one cationic membrane; or
one bipolar membrane; or
one anionic membrane and one cationic membrane; or
one cationic membrane and one bipolar membrane; or
one anionic membrane and one bipolar membrane; or
one anionic membrane, one cationic membrane and one bipolar membrane.

8. Method of any one of the preceding claims, further comprising step (Y):
(Y) concentrating or concentrating and drying the product obtained according to the method defined in any one of claims 1 to 7.

9. Composition consisting of an alkali metal salt of a compound of the general formula (I) or formula (II)
R¹R²N-CHR³-COOH or R¹R²N-CHR³CHR³-COOH (I) (II),
wherein R¹, R² and R³ are independently H, an alkyl, an alkenyl, or a hydroxyalkyl radical containing from 1 to 18 carbon atoms, or from 1 to 6 carbon atoms; or wherein R¹ and R² form jointly together with the N atom a heterocyclic 5- or 6-membered ring;
and an alkali metal hydroxide in a quantity of less than 0.5 % or not more than 0.1 % by weight, based on the weight of the alkali metal salt of the compound of the general formula (I) or formula (II) and, if any is present, the alkali metal hydroxide; and
a chloride content of not more than 75 ppm and a sulfate content of not more than 750 ppm, each based on the total weight of the composition; wherein the composition contains N-methylglycine present in an amount of at least 0.1 wt.% and up to 0.3 wt%, based on the total weight of the composition.

10. The composition of claim 9, wherein the chloride content does not exceed 50 ppm; or wherein the sulfate content does not exceed 100 ppm.

11. Composition of claim 9 obtained by a method as defined in any one of claims 1 to 7.

12. Use of a composition as defined in any one of claims 9 to 10or claim 11, as feed additive.

13. Use of the composition of claim 12, wherein said feed additive is for poultry in case of an alkali metal salt of the compound of formula (I), and for pig in case of an alkali metal salt of the compound of formula (II).

## Patentansprüche

1. Verfahren zur Stabilisierung eines Alkalimetallsalzes einer Verbindung der allgemeinen Formel (I) oder Formel (II)
R¹R²N-CHR³-COOH oder R¹R²N-CHR³CHR³-COOH (I) (II)
wobei R¹, R² und R³ unabhängig voneinander H, Alkyl, Alkenyl, oder Hydroxyalkylradikal, enthaltend 1 bis 18 Kohlenstoffatome, oder von 1 bis 6 Kohlenstoffatome, sind; oder wobei R¹ und R² gemeinsam zusammen mit dem N-Atom einen heterozyklischen 5- oder 6-gliedrigen Ring bilden;
besagtes Verfahren aufweisend Bereitstellen einer Mischung aufweisend besagtes Salz, Alkalimetallhydroxid und Wasser, und Durchführen eines Schrittes (X) mit der Mischung:
(X) Reduzieren der Menge des besagten Alkalimetallhydroxids in der Mischung wobei Schritt (X) aufweist
(A) Reduzieren der Menge des besagten Alkalimetallhydroxids in der Mischung durch Aussetzen der Mischung gegenüber Elektrodialyse; oder
(B) Reduzieren der Menge des besagten Alkalimetallhydroxids in der Mischung durch Hinzugeben einer Säure zu der Mischung und (A) Reduzieren der Menge des besagten Alkalimetallhydroxids in der Mischung durch Aussetzen der Mischung gegenüber Elektrodialyse;
wobei in Schritt (A) besagte Elektrodialyse durchgeführt wird in einer Elektrodialysezelle, deren Wiederholungseinheit besteht aus
einer anionischen Membran und einer kationischen Membran und zwei Kompartimenten; oder
einer bipolaren Membran und einer kationischen Membran und zwei Kompartimenten; oder
einer bipolaren Membran und einer anionischen Membran und zwei Kompartimenten; oder
einer bipolaren Membran, einer anionischen Membran und einer kationischen Membran und drei Kompartimenten; oder
einer anionischen Membran und einer kationischen Membran und zwei Kompartimenten, wobei besagtes Alkalimetallhydroxid umfasst in besagter Mischung neutralisiert wird durch Hinzugeben einer Säure vor dem Aussetzen besagter Mischung gegenüber Elektrodialyse;
zwei kationischen Membranen in einem zwei Kompartimente-Arrangement, und wobei besagtes Alkalimetallhydroxid umfasst in besagter Mischung neutralisiert wird durch Hinzugeben einer Säure simultan zum Aussetzen besagter Mischung gegenüber Elektrodialyse; oder
zwei angrenzenden kationischen Membranen, einer anionischen Membran und drei Kompartimenten, und wobei besagtes Alkalimetallhydroxid umfasst in besagter Mischung neutralisiert wird durch Hinzugeben einer Säure simultan zum Aussetzen besagter Mischung gegenüber Elektrodialyse; oder
zwei angrenzenden anionischen Membranen, einer kationischen Membran und drei Kompartimenten, und wobei besagtes Alkalimetallhydroxid umfasst in besagter Mischung neutralisiert wird durch Hinzugeben einer Säure simultan zum Aussetzen besagter Mischung gegenüber Elektrodialyse;
wobei die Säure hinzugegeben in Schritt (B) eine Verbindung der Formel (I) oder Formel (II) ist; oder wobei die Säure hinzugegeben in Schritt (B) erhalten wird durch Aussetzen mindestens eines Teils der besagten Mischung gegenüber einem lonenaustausch nutzend einen azidischen lonenaustauscher.

2. Verfahren zum Aufreinigen eines Alkalimetallsalzes einer Verbindung der allgemeinen Formel (I) oder Formel (II)
R¹R²N-CHR³-COOH oder R¹R²N-CHR³CHR³-COOH (I) (II)
wobei R¹, R² und R³ unabhängig voneinander H, Alkyl, Alkenyl, oder Hydroxyalkylradikal, umfassend von 1 bis 18 Kohlenstoffatome, oder von 1 bis 6 Kohlenstoffatome, sind; oder wobei R¹ und R² gemeinsam zusammen mit dem N-Atom einen heterozyklischen 5- oder 6-gliedrigen Ring bilden;
besagtes Verfahren aufweisend Bereitstellen einer Mischung aufweisend besagtes Salz, ein Alkalimetallhydroxid und Wasser, und Aussetzen der Mischung gegenüber einem Schritt (X):
(X) Reduzieren der Menge des besagten Alkalimetallhydroxids in der Mischung;
wobei
(A) wobei besagtes Alkalimetallhydroxid reduziert wird durch Aussetzen besagter Mischung gegenüber Elektrodialyse; oder
(B) besagtes Alkalimetallhydroxid reduziert wird durch Hinzugeben einer Säure zu besagter Mischung und (A) besagtes Alkalimetall reduziert wird durch Aussetzen besagter Mischung gegenüber Elektrodialyse;
wobei in Schritt (A) besagte Elektrodialyse durchgeführt wird in einer Elektrodialysezelle, deren Wiederholungseinheit besteht aus
einer anionischen Membran und einer kationischen Membran und zwei Kompartimenten; oder
einer bipolaren Membran und einer kationischen Membran und zwei Kompartimenten; oder
einer bipolaren Membran und einer anionischen Membran und zwei Kompartimenten; oder
einer bipolaren Membran, einer anionischen Membran und einer kationischen Membran und drei Kompartimenten; oder
einer anionischen Membran und einer kationischen Membran und zwei Kompartimenten, wobei besagtes Alkalimetallhydroxid umfasst in besagter Mischung neutralisiert wird durch Hinzugeben einer Säure vor dem Aussetzen besagter Mischung gegenüber Elektrodialyse;
zwei kationischen Membranen in einem zwei Kompartimente-Arrangement, und wobei besagtes Alkalimetallhydroxid umfasst in besagter Mischung neutralisiert wird durch Hinzugeben einer Säure simultan zum Aussetzen besagter Mischung gegenüber Elektrodialyse; oder
zwei angrenzenden kationischen Membranen, einer anionischen Membran und drei Kompartimenten, und wobei besagtes Alkalimetallhydroxid umfasst in besagter Mischung neutralisiert wird durch Hinzugeben einer Säure simultan zum Aussetzen besagter Mischung gegenüber Elektrodialyse; oder
zwei angrenzenden anionischen Membranen, einer kationischen Membran und drei Kompartimenten, und wobei besagtes Alkalimetallhydroxid umfasst in besagter Mischung neutralisiert wird durch Hinzugeben einer Säure simultan zum Aussetzen besagter Mischung gegenüber Elektrodialyse;
wobei die Säure hinzugegeben in Schritt (B) eine Verbindung der Formel (I) oder Formel (II) ist; oder wobei die Säure hinzugegeben in Schritt (B) erhalten wird durch Aussetzen mindestens eines Teils der besagten Mischung gegenüber einem lonenaustausch nutzend einen azidischen lonenaustauscher.

3. Verfahren nach irgendeinem der vorangegangenen Ansprüche, wobei besagtes Alkalimetallsalz der Verbindung nach Formel (I) oder Formel (II) hergestellt wird in einem Verfahren zum Herstellen einer Verbindung nach Formel (I) oder Formel (II) in Gegenwart von einem Alkalimetallhydroxid; oder wobei besagtes Alkalimetallsalz der Verbindung nach Formel (I) oder Formel (II) hergestellt wird durch In-Kontakt-bringen der Verbindung nach Formel (I) oder Formel (II) mit einem Alkalimetallhydroxid.

4. Verfahren nach irgendeinem der vorangegangenen Ansprüche, wobei
in Verbindung nach Formel (I) R¹ = R² = CH₃ und R³ = H; oder
in Verbindung nach Formel (II) R¹ = R² = R³ = H.

5. Verfahren nach irgendeinem der vorangegangenen Ansprüche, wobei besagtes Alkalimetall Natrium aufweist oder Natrium ist.

6. Verfahren nach irgendeinem der vorangegangenen Ansprüche, wobei besagte Mischung besagtes Alkalimetallhydroxid in einer Menge von 0.05 bis 5 Gewicht% aufweist, basierend auf dem Gewicht der Mischung; oder wobei besagte Mischung besagtes Alkalimetallsalz der besagten Verbindung nach Formel (I) oder Formel (II) in einer Menge von 5 bis 45 Gew.%, basierend auf dem Gewicht der Mischung aufweist.

7. Verfahren nach irgendeinem der Ansprüche 1 bis 6, wobei in Schritt (A) besagte Elektrodialyse durchgeführt wird in einer Elektrodialysezelle aufweisend mindestens
eine anionische Membran; oder
eine kationische Membran; oder
eine bipolare Membran; oder
eine anionische und eine kationische Membran; oder
eine kationische und eine bipolare Membran; oder
eine anionische Membran und eine bipolare Membran; oder
eine anionische Membran, eine kationische Membran und eine bipolare Membran.

8. Verfahren nach irgendeinem der vorangegangenen Ansprüche, weiterhin aufweisend Schritt (Y):
(Y) Konzentrieren oder Konzentrieren und Trocknen des Produktes erhalten nach dem Verfahren definiert in irgendeinem der Ansprüche 1 bis 7.

9. Zusammensetzung bestehend aus einem Alkalimetallsalz einer Verbindung der allgemeinen Formel (I) oder Formel (II)
R¹R²N-CHR³-COOH oder R¹R²N-CHR³CHR³-COOH (I) (II)
wobei R¹, R² und R³ unabhängig voneinander H, Alkyl, Alkenyl, oder Hydroxyalkylradikal, umfassend von 1 bis 18 Kohlenstoffatome, oder von 1 bis 6 Kohlenstoffatome, sind; oder wobei R¹ und R² gemeinsam zusammen mit dem N-Atom einen heterozyklischen 5- oder 6-gliedrigen Ring bilden;
und ein Alkalimetallhydroxid in einer Menge von weniger als 0.5% oder nicht mehr als 0.1 Gewicht%, basierend auf dem Gewicht des Alkalimetallsalzes der Verbindung der allgemeinen Formel (I) oder Formel (II) und, sofern welches vorhanden ist, das Alkalimetallhydroxid; und
einen Chloridgehalt von nicht mehr als 75 ppm und einen Sulfatgehalt von nicht mehr als 750 ppm, jeweils basierend auf dem Gesamtgewicht der Zusammensetzung; wobei die Zusammensetzung N-Methylglycin umfasst, vorhanden in einer Menge von mindestens 0.1 Gew.% und bis zu 0.3 Gew.%, basierend auf dem Gesamtgewicht der Zusammensetzung.

10. Die Zusammensetzung nach Anspruch 9, wobei der Chloridgehalt 50 ppm nicht überschreitet; oder wobei der Sulfatgehalt 100 ppm nicht überschreitet.

11. Zusammensetzung nach Anspruch 9 erhalten durch ein Verfahren wie definiert in irgendeinem der Ansprüche 1 bis 7.

12. Verwendung einer Zusammensetzung wie definiert in irgendeinem der Ansprüche 9 bis 10 oder Anspruch 11, als Futtermittelzusatzstoff.

13. Verwendung der Zusammensetzung nach Anspruch 12, wobei besagter Futtermittelzusatzstoff für Geflügel im Fall eines Alkalimetallsalzes der Verbindung nach Formel (I), und für Schweine im Fall eines Alkalimetallsalzes der Verbindung nach Formel (II) ist.

## Revendications

1. Procédé de stabilisation d'un sel de métal alcalin d'un composé de formule générale (I) ou de formule générale (II)
R¹R²N-CHR³-COOH ou R¹R²N-CHR³-CHR³-COOH (I) (II),
dans lequel R¹, R² et R³ sont indépendamment H, un alkyle, un alcényle ou un radical hydroxyalkyle ayant de 1 à 18 atomes de carbone, ou de 1 à 6 atomes de carbone ; ou dans lequel R¹ et R² forment conjointement avec l'atome N un noyau hétérocyclique de 5 ou 6 chaînons ;
ledit procédé comprenant la fourniture d'un mélange comprenant ledit sel, un hydroxyde de métal alcalin et de l'eau, et la soumission du mélange à une étape (X) consistant à :
(X) réduire la quantité dudit hydroxyde de métal alcalin dans le mélange dans lequel l'étape (X) comprend
(A) la réduction de la quantité dudit hydroxyde de métal alcalin dans le mélange en soumettant le mélange à une électrodialyse ; ou
(B) la réduction de la quantité dudit hydroxyde de métal alcalin dans le mélange en ajoutant un acide au mélange et (A) la réduction de la quantité dudit hydroxyde de métal alcalin dans le mélange en soumettant le mélange à une électrodialyse ;
dans lequel dans l'étape (A), ladite électrodialyse est effectuée dans une cellule d'électrodialyse dont l'unité de répétition consiste en
une membrane anionique et une membrane cationique et deux compartiments ; ou
une membrane bipolaire et une membrane cationique et deux compartiments ; ou
une membrane bipolaire et une membrane anionique et deux compartiments ; ou
une membrane bipolaire, une membrane anionique et une membrane cationique et trois compartiments ; ou
une membrane anionique et une membrane cationique et deux compartiments, dans lequel ledit hydroxyde de métal alcalin compris dans ledit mélange est neutralisé par ajout d'un acide avant soumission dudit mélange à une électrodialyse ; ou
deux membranes cationiques dans un agencement à deux compartiments, et dans lequel ledit hydroxyde de métal alcalin compris dans ledit mélange est neutralisé par ajout d'un acide simultanément à la soumission dudit mélange à une électrodialyse ; ou
deux membranes cationiques adjacentes, une membrane anionique et trois compartiments, et dans lequel ledit hydroxyde de métal alcalin compris dans ledit mélange est neutralisé par ajout d'un acide simultanément à la soumission dudit mélange à une électrodialyse ; ou
deux membranes anioniques adjacentes, une membrane cationique et trois compartiments, et dans lequel ledit hydroxyde de métal alcalin compris dans ledit mélange est neutralisé par ajout d'un acide simultanément à la soumission dudit mélange à une électrodialyse ;
dans lequel l'acide ajouté à l'étape (B) est un composé de formule (I) ou de formule (II) ; ou dans lequel l'acide ajouté à l'étape (B) est obtenu par soumission d'au moins une partie dudit mélange à un échange d'ions à l'aide d'un échangeur d'ions acides.

2. Procédé de purification d'un sel de métal alcalin d'un composé de formule générale (I) ou de formule générale (II)
R¹R²N-CHR³-COOH ou R¹R²N-CHR³-CHR³-COOH (I) (II),
dans lequel R¹, R² et R³ sont indépendamment H, un alkyle, un alcényle ou un radical hydroxyalkyle ayant de 1 à 18 atomes de carbone, ou de 1 à 6 atomes de carbone ; ou dans lequel R¹ et R² forment conjointement avec l'atome N un noyau hétérocyclique de 5 ou 6 chaînons ;
ledit procédé comprenant la fourniture d'un mélange comprenant ledit sel, un hydroxyde de métal alcalin et de l'eau, et la soumission du mélange à une étape (X) consistant à :
(X) réduire la quantité dudit hydroxyde de métal alcalin dans le mélange ;
dans lequel
(A) ledit hydroxyde de métal alcalin est réduit par soumission dudit mélange à une électrodialyse ;ou
(B) ledit hydroxyde de métal alcalin est réduit par ajout d'un acide audit mélange et (A) ledit hydroxyde de métal alcalin est réduit par soumission dudit mélange à une électrodialyse ;
dans lequel dans l'étape (A), ladite électrodialyse est effectuée dans une cellule d'électrodialyse dont l'unité de répétition consiste en
une membrane anionique et une membrane cationique et deux compartiments ; ou
une membrane bipolaire et une membrane cationique et deux compartiments ; ou
une membrane bipolaire et une membrane anionique et deux compartiments ; ou
une membrane bipolaire, une membrane anionique et une membrane cationique et trois compartiments ; ou
une membrane anionique et une membrane cationique et deux compartiments, dans lequel ledit hydroxyde de métal alcalin compris dans ledit mélange est neutralisé par ajout d'un acide avant soumission dudit mélange à une électrodialyse ; ou
deux membranes cationiques dans un agencement à deux compartiments, et dans lequel ledit hydroxyde de métal alcalin compris dans ledit mélange est neutralisé par ajout d'un acide simultanément à la soumission dudit mélange à une électrodialyse ; ou
deux membranes cationiques adjacentes, une membrane anionique et trois compartiments, et dans lequel ledit hydroxyde de métal alcalin compris dans ledit mélange est neutralisé par ajout d'un acide simultanément à la soumission dudit mélange à une électrodialyse ; ou
deux membranes anioniques adjacentes, une membrane cationique et trois compartiments, et dans lequel ledit hydroxyde de métal alcalin compris dans ledit mélange est neutralisé par ajout d'un acide simultanément à la soumission dudit mélange à une électrodialyse ;
dans lequel l'acide ajouté à l'étape (B) est un composé de formule (I) ou de formule (II) ; ou dans lequel l'acide ajouté à l'étape (B) est obtenu par soumission d'au moins une partie dudit mélange à un échange d'ions à l'aide d'un échangeur d'ions acides.

3. Procédé selon l'une quelconque des revendications précédentes, dans lequel ledit sel de métal alcalin du composé de formule (I) ou de formule (II) est préparé dans un procédé de fabrication d'un composé de formule (I) ou de formule (II) en présence d'un hydroxyde de métal alcalin ; ou dans lequel ledit sel de métal alcalin du composé de formule (I) ou de formule (II) est préparé par mise en contact du composé de formule (I) ou de formule (II) avec un hydroxyde de métal alcalin.

4. Procédé selon l'une quelconque des revendications précédentes, dans lequel
dans le composé de formule (I) R¹ = R² = CH₃ et R³ = H ; ou
dans le composé de formule (II) R¹ = R² = R³ = H.

5. Procédé selon l'une quelconque des revendications précédentes, dans lequel ledit métal alcalin comprend du sodium ou est du sodium.

6. Procédé selon l'une quelconque des revendications précédentes, dans lequel ledit mélange comprend ledit hydroxyde de métal alcalin en une quantité de 0,05 à 5 % en poids, sur la base du poids du mélange ; ou dans lequel ledit mélange comprend ledit sel de métal alcalin dudit composé de formule (I) ou de formule (II) en une quantité de 5 à 45 % en poids, sur la base du poids du mélange.

7. Procédé selon l'une quelconque des revendications 1 à 6, dans lequel, à l'étape (A), ladite électrodialyse est effectuée dans une cellule d'électrodialyse comprenant au moins
une membrane anionique ; ou
une membrane cationique ; ou
une membrane bipolaire ; ou
une membrane anionique et une membrane cationique ; ou
une membrane cationique et une membrane bipolaire ; ou
une membrane anionique et une membrane bipolaire ; ou
une membrane anionique, une membrane cationique et une membrane bipolaire.

8. Procédé selon l'une quelconque des revendications précédentes, comprenant en outre l'étape (Y) :
(Y) concentration ou concentration et séchage du produit obtenu selon le procédé défini dans l'une quelconque des revendications 1 à 7.

9. Composition constituée d'un sel de métal alcalin d'un composé de formule générale (I) ou de formule générale (II)
R¹R²N-CHR³-COOH ou R¹R²N-CHR³-CHR³-COOH (I) (II),
dans lequel R¹, R² et R³ sont indépendamment H, un alkyle, un alcényle ou un radical hydroxyalkyle ayant de 1 à 18 atomes de carbone, ou de 1 à 6 atomes de carbone ; ou dans lequel R¹ et R² forment conjointement avec l'atome N un noyau hétérocyclique de 5 ou 6 chaînons ;
et un hydroxyde de métal alcalin en une quantité inférieure à 0,5 % ou inférieure ou égale à 0,1 % en poids, sur la base du poids du sel de métal alcalin du composé de formule générale (I) ou de formule générale (II) et, le cas échéant, de l'hydroxyde de métal alcalin ; et
une teneur en chlorure inférieure ou égale à 75 ppm et une teneur en sulfate inférieure ou égale à 750 ppm, chacune basée sur le poids total de la composition ; dans laquelle la composition contient de la N-méthylglycine présente en une quantité d'au moins 0,1 % en poids et jusqu'à 0,3 % en poids, sur la base du poids total de la composition.

10. Composition selon la revendication 9, dans laquelle la teneur en chlorure ne dépasse pas 50 ppm ; ou dans laquelle la teneur en sulfate ne dépasse pas 100 ppm.

11. Composition selon la revendication 9 obtenue par le procédé tel que défini dans l'une quelconque des revendications 1 à 7.

12. Utilisation d'une composition telle que définie dans l'une quelconque des revendications 9 à 10 ou la revendication 11, en tant qu'additif alimentaire.

13. Utilisation de la composition selon la revendication 12, dans laquelle ledit additif alimentaire est destiné à la volaille dans le cas d'un sel de métal alcalin du composé de formule (I), et au porc dans le cas d'un sel de métal alcalin du composé de formule (II).
